# EUROPÄISCHE PATENTANMELDUNG

(11) **EP 3 075 703 A1**
(43) Veröffentlichungstag der Anmeldung: **05.10.2016**
(21) Anmeldenummer: 16000386.9
(22) Anmeldetag: 16.02.2016
(51) Int. Cl.: C01B 3/38, C01B 3/48

(54) **VERFAHREN ZUR ERZEUGUNG VON SYNTHESEGAS AUS EINEM CO2-REICHEN, KOHLENWASSERSTOFFHALTIGEN EINSATZGAS**

(30) Priorität: 31.03.2015 DE 102015004214
(71) Anmelder: Linde Aktiengesellschaft, 80331 München (DE)
(72) Erfinder: Ranke, Harald, 82343 Pöcking (DE); Guzmann, Marcus, 82541 Münsing (DE); Mabrouk, Rachid, 81477 München (DE); Heucke, Ulrich, 81247 München (DE)
(74) Vertreter: Fischer, Werner

(57) **Zusammenfassung**

Die Erfindung betriff ein Verfahren zur Erzeugung eines Synthesegases aus einem CO₂-reichen und kohlenwasserstoffhaltigen Einsatzgas (NG), wobei ein CO₂-reiches und kohlenwasserstoffhaltiges Einsatzgas (NG) bereitgestellt wird und in einem Synthesegaserzeugungsschritt (50) mittels partieller Oxidation (50) und/oder Dampfreformierung (50) zu einem H₂ und CO aufweisenden Synthesegas umgesetzt wird. Erfindungsgemäß ist vorgesehen, dass zumindest CO₂ aus dem Einsatzgas (NG) bei einer Wäsche (10) des Einsatzgases (NG) mittels eines Waschmittels entfernt wird, bevor das Einsatzgas (NG) dem Synthesegaserzeugungsschritt (50) zugeführt wird.

## Beschreibung

Die Erfindung betrifft ein Verfahren zur Erzeugung eines Synthesegases aus einem kohlenwasserstoffhaltigen Einsatzgas gemäß Anspruch 1.

Hierbei wird ein kohlenwasserstoffhaltiges Einsatzgas, das Methan aufweist, bereitgestellt und in einem Synthesegaserzeugungsschritt mittels partieller Oxidation und/oder Dampfreformierung zu einem H₂ und CO aufweisenden Synthesegas umgesetzt.

Derartige Verfahren sind z.B. aus der US2012/0326090 A1, US2012/0282145 A1, US2013/008536233 B2 bekannt.

Derzeit sind weltweit ausreichende Gasvorkommen als Einsatz für die Synthesegasherstellung vorhanden, wobei jedoch die Gasqualität, insbesondere im Hinblick auf die Zusammensetzung signifikant variiert. Damit einhergehend ist die Verwendbarkeit dieser natürlichen Gasvorkommen eingeschränkt. Insbesondere niedrigkalorische Gase und/oder Gase mit einem hohen Gehalt an inerten Bestandteilen wie z.B. CO₂ lassen sich heute nur schwer oder sogar gar nicht verwenden, da Verarbeitungsprozesse nach dem Stand der Technik oft nicht ökonomisch sind.

Die Umwandlung eines Einsatzgases mit einem hohen Inertengehalt in chemische Produkte verlangt eine vergleichsweise höhere Einsatzgasmenge, so dass die Kosten für Apparate und der Energiebedarf für das Betreiben von Apparaten sowie für die Kühlung und das Erhitzen von Prozessströmen entsprechend steigen.

Der Erfindung liegt vor diesem Hintergrund die Aufgabe zugrunde, ein Verfahren der eingangs genannten Art zu verbessern.

Diese Aufgabe wird durch ein Verfahren mit den Merkmalen des Anspruchs 1 gelöst.

Danach ist erfindungsgemäß vorgesehen, dass zumindest CO₂ aus dem Einsatzgas bei einer Wäsche des Einsatzgases mittels eines Waschmittels (z.B. Lösungsmittel) entfernt wird, bevor das Einsatzgas dem Synthesegaserzeugungsschritt zugeführt wird. Unter einem CO₂-reichen Einsatzgas wird vorliegend ein Einsatzgas verstanden, dass einen CO₂-Gehalt von zumindest 10 Vol.-%, 20 Vol.-%, 30 Vol.-%, 40 Vol.-%, 50 oder zumindest 60 Vol.-% aufweist.

Bei der besagten Wäsche wird bevorzugt das CO₂ physikalisch im Waschmittel, bei dem es sich um Methanol oder Dimethylether (DME) handeln kann, gelöst. Weiterhin kann das Waschmittel Methanol und/oder DME aufweisen.

Bevorzugt wird also bei der Wäsche ein kaltes, methanolhaltiges Waschmittel als physikalisches Lösungsmittel zur Abtrennung von CO₂ aus dem Einsatzgasstrom verwendet. Der Einsatzgasstrom wird dabei mit dem Waschmittel kontaktiert, wobei CO₂ in dem Waschmittel physikalisch gelöst wird. Da die physikalische Löslichkeit der gasförmigen Komponenten des Einsatzgases im Waschmittel bei sinkender Temperatur zunimmt, erfolgt die CO₂-Absorption im Waschmittel bevorzugt bei niedrigen Temperaturen im Bereich von ca. -35°C bis -65°C sowie vorzugsweise bei einem Druck im Bereich von 20 bar bis 60 bar.

Aufgrund der unterschiedlichen Löslichkeit der ggf. weiteren Komponenten des Einsatzgases (insbesondere Schwefelverbindungen) in dem vorzugsweise methanolhaltigen Waschmittel ist es bei der besagten Wäsche möglich, CO₂ getrennt von einer oder mehreren weiteren Komponenten des Einsatzgases abzutrennen. Bei einer weiteren Komponente des Einsatzgases kann es sich z.B. um Schwefelverbindungen wie bspw. H₂S, CS₂, COS und/oder HCN handeln. Derart isolierte Nebenprodukte können somit ebenfalls separat weiterverwendet werden.

Insbesondere wird bei der erfindungsgemäßen Wäsche das Einsatzgas in eine Adsorptionskolonne geleitet und mit dem vorzugsweise methanolhaltigen Waschmittel in Kontakt gebracht, z.B. im Gegenstrom.

Aufgrund der unterschiedlichen Löslichkeitskoeffizienten der einzelnen Komponenten gegenüber dem Waschmittel erfolgt eine Anreicherung einzelner Komponenten in bestimmten Bereichen innerhalb der Absorptionkolonne. Beispielsweise weist die Absorptionskolonne einen ersten Abschnitt mit einem erhöhten Anteil an Schwefelkomponenten (v.a. H₂S und COS) auf. Weiterhin weist die Absorptionskolonne einen zweiten Abschnitt mit einem erhöhten Anteil an CO₂ auf. Schließlich weist die Absorptionskolonne einen dritten Abschnitt auf, in welchem im Wesentlichen das von CO₂ und ggf. den besagten Schwefelverbindungen befreite Einsatzgas vorliegt. Das Waschmittel in der Absorptionseinrichtung weist dabei bevorzugt eine Temperatur sowie einen Druck in den oben genannten Bereichen auf.

Bevorzugt wird aus dem dritten Abschnitt das Einsatzgas abgezogen und der Synthesegaserzeugung zugeführt.

Das mit CO₂ beladene Waschmittel wird vorzugsweise aus dem zweiten Abschnitt der Adsorptionskolonne in eine Desorptionskolonne gefahren. In der Desorptionskolonne wird das CO₂ mittels einer Entspannung (die Löslichkeit der einzelnen Komponenten sinkt bei niedrigerem Druck) vom Waschmittel entfernt und dabei auch von den ggf. vorhandenen weiteren Sauergaskomponenten, welche noch im Waschmittel gelöst sind (z.B. H₂S und COS), abgetrennt. Alternativ oder zusätzlich kann das CO₂ in der Desorptionskolonne durch Einleiten eines Strippgases (z.B. N₂) vom Waschmittel abgetrennt werden. Das abgetrennte CO₂ sammelt sich dabei in einem entsprechenden Abschnitt der Desorptionskolonne und kann von dort abgezogen werden.

Bevorzugt wird der z. B. solchermaßen erzeugte CO₂-reiche Strom, der vorzugsweise zumindest einen CO₂-Gehalt von 99 Vol.-% aufweist, und der das abgetrennte CO₂ enthält, bei einem Druck (abhängig vom Druck des Einsatzgases) im Bereich von vorzugsweise 10 bar bis 100 bar bereitgestellt und vorzugsweise einer weiteren Verwendung zugeführt.

Vorzugsweise (siehe auch unten) wird dieser CO₂-reiche Strom als Einsatz für eine Synthese verwendet, insbesondere für eine Methanolsynthese, z.B. katalytisch gemäß

CO₂ + 3H₂ <-> CH₃OH + H₂O.

Alternativ oder ergänzend wird der CO₂-reiche Strom gemäß einer Ausführungsform der Erfindung zur Unterstützung der Förderung von Erdöl ("Enhanced Oil Recovery" oder kurz EOR) verwendet, wobei der CO₂-reiche Strom in eine Erdöl-Lagerstätte injiziert wird, um die Ölproduktionsraten bzw. -ausbeute zu erhöhen, z.B. durch Erhöhung des Lagerstättendrucks. Weiterhin kann CO₂ auch als Zusatz eines Flutmediums verwendet werden, das in die Erdöllagerstätte eingeleitet wird.

In der Desorptionskolonne bildet sich des Weiteren ein weiterer Abschnitt, in welchem im Wesentlichen (soweit vorhanden) die besagten Schwefelkomponenten in dem Waschmittel gelöst sind.

Des Weiteren kann das Waschmittel aus dem weiteren Abschnitt der Absorptionskolonne, welcher einen erhöhten Anteil an Schwefelkomponenten aufweist, in die Desorptionskolonne geleitet werden, so dass eventuell vorhandenes CO₂ aus dem mit Schwefelkomponenten angereicherten Waschmittel entfernt werden kann.

Das Waschmittel aus dem weiteren Abschnitt der Desorptionskolonne, welches im Wesentlichen jene Schwefelkomponenten aufweist, wird aus dem weiteren Abschnitt bevorzugt in eine Heißregenerationskolonne, in der eine Entfernung der noch im Waschmittel enthaltenen Schwefelkomponenten mittels eines Erhitzens des Waschmittels erfolgt. Der so erhaltene Gasstrom mit den Schwefelkomponenten kann dann einer weiteren Verwendung zugeführt werden.

Das vorstehend beschriebene Waschverfahren unter Verwendung von Methanol als Waschmittel wird auch als Rectisolverfahren bezeichnet.

Gemäß einer weiteren Ausführungsform der Erfindung ist vorgesehen, dass der solchermaßen von CO₂ und ggf. weiteren Komponenten befreite Einsatzgasstrom stromab der besagten Wäsche durch eine Adsorbereinheit geführt wird, wobei eine oder mehrere noch im Einsatzgas befindliche Schwefelverbindungen in der Adsorbereinheit adsorbiert und dabei aus dem Einsatzgas entfernt werden.

Nach der besagten Wäsche weist der Einsatzgasstrom bevorzugt lediglich noch einen Gehalt von bis zu 1000 ppm CO₂ auf. Die oben genannten Schwefelverbindungen liegen nach der besagten Wäsche bevorzugt jeweils lediglich noch mit einem Gehalt von bis zu 1000 ppm im Einsatzgasstrom vor.

Die Adsorbereinheit stromab der Wäsche dient insbesondere dazu, die noch im Einsatzgas vorhandenen geringen Konzentrationen der ungewünschten Verbindungen weiter zu verringern, so dass bevorzugt CO₂ und ggf. die besagten Schwefelverbindung jeweils noch mit einem Gehalt von höchstens 10 ppm im Einsatzgas vorliegen.

In dem Synthesegaserzeugungsschritt kann zur Synthesegaserzeugung, wie eingangs erwähnt, die partielle Oxidation (POX) und/oder die Dampfreformation verwendet werden.

Der Einsatzgasstrom weist bevorzugt einen oder mehrere der folgenden Komponenten bzw. Kohlenwasserstoffe auf, die im Synthesegaserzeugungsschritt zu dem H₂ und CO umfassenden Synthesegas umgesetzt werden: CH₄, H₂O, CO₂
Bei der partiellen Oxidation wird der wie oben beschrieben vorgereinigte Einsatzgasstrom, der z. B. Erdgas bzw. CH₄ aufweist, substöchiometrisch in einem exothermen Prozess umgesetzt. Reaktionsprodukte sind vor allem Wasserstoff und Kohlenstoffmonoxid, die gemäß

CₙHₘ + n/2 O₂ -> n CO + m/2 H₂

erhalten werden.

Bei der Dampfreformation wird der wie oben beschrieben vorgereinigte Einsatzgasstrom, der z.B. Erdgas bzw. CH₄ aufweist, mit überhitztem Prozess- bzw. Wasserdampf entsprechend einem für das Reformieren ausreichenden Dampf/Kohlenstoff-Verhältnis gemischt. Anschließend wird dieses Gasgemisch aufgeheizt und auf die mit einem Katalysator gefüllten Reaktorrohre des verwendeten Ofens bzw. Reformers verteilt. Das Gemisch strömt bevorzugt von oben nach unten durch die in vertikalen Reihen angeordneten Reaktorrohre. Beim Durchströmen der vorzugsweise von außen befeuerten Reaktorrohre reagiert das Kohlenwasserstoff/Dampf-Gemisch unter Bildung von Wasserstoff und Kohlenmonoxid z.B. gemäß:

CₙHₘ + n H₂O => n CO + (n+m)/2 H₂ (1)

CH₄ + H₂O <=> CO+ 3H₂ (2)

CO + H₂O <=> CO₂+ H₂ (3).

Da die Wärmebilanz für die Hauptreaktionen (1) - (2) endotherm ist, wird die benötigte Wärme durch einen Verbrennungsprozess im verwendeten Ofen zugeführt.

Gemäß einer bevorzugten Ausführungsform der Erfindung ist vorgesehen, dass das erzeugte Synthesegas in einen ersten und einen zweiten Synthesegasteilstrom aufgeteilt wird, wobei der erste Synthesegasteilstrom als Einsatz für eine Synthese verwendet wird, und wobei der zweite Synthesegasteilstrom einer Wassergas-Shift-Reaktion gemäß

CO + H₂O <-> CO₂ + H₂

unterzogen wird, wobei CO des zweiten Synthesegasteilstromes mit H₂O zu H₂ und CO₂ umgesetzt wird, um den CO-Gehalt im zweiten Synthesegasteilstrom zu verringern und den Wasserstoffgehalt im zweiten Synthesegasteilstrom zu erhöhen.

Bevorzugt wird die Reduktion des CO₂-Gehalts im Einsatzgas bei der Wäsche in Abhängigkeit von einer stromab der Synthesegaserzeugung vorgesehenen Verwendung des Synthesegases und/oder in Abhängigkeit eines gewünschten Verhältnisses von CO zu H₂ im Synthesegas eingestellt.

Gemäß einer bevorzugten Ausführungsform der Erfindung ist weiterhin vorgesehen, dass der zweite Synthesegasteilstrom nach der Wassergas-Shift-Reaktion einer Druckwechseladsorption unterzogen wird, wobei im zweiten Synthesegasteilstrom enthaltenes CO₂ sowie ferner auch darin enthaltenes CO, H₂, CH₄ und/oder H₂O an einem Adsorber bei einem ersten Druck adsorbiert wird und ein H₂-haltiger Strom erzeugt wird, und wobei der Adsorber bei einem zweiten Druck, der niedriger ist als der erste Druck, regeneriert wird, wobei adsorbiertes CO₂ desorbiert wird, und wobei der Adsorber zum Entfernen des desorbierten CO₂ mit einem H₂-haltigen Spülgasstrom gespült wird.

Bevorzugt wird dieser Spülgasstrom als Brennstoff verwendet, z.B. zur Bereitstellung von Wärme in einem Ofen zur Durchführung der (oben beschriebenen) Dampfreformation. Alternativ hierzu, z.B. bei Verwendung der POX (siehe oben), kann das Spülgas auch in einer sonstigen Verbrennungseinrichtung verbrannt werden, z.B. zum Erzeugen und/oder Überhitzen von Wasser- bzw. Prozessdampf.

Sofern in dem Synthesegaserzeugungsschritt die partielle Oxidation verwendet wird (siehe oben) wird gemäß einer bevorzugten Ausführungsform der Erfindung Sauerstoff aus Luft abgetrennt (z.B. in einer kryogenen Luftzerlegungsanlage) und als Oxidationsmittel bei der partiellen Oxidation verwendet, wobei der Sauerstoff bzw. Oxidationsmittelstrom dem Einsatzgas stromab der Wäsche, stromab der Adsorbereinheit sowie stromauf des Synthesegaserzeugungsschritts dem Einsatzgas zugegeben wird. Vorzugsweise wird reiner Sauerstoff als Oxidationsmittel verwendet, der lediglich Verunreinigungen unterhalb von 5 Vol.-% aufweist.

Gemäß einer bevorzugten Ausführungsform der Erfindung ist vorgesehen, dass der erste Synthesegasteilstrom in einer Fischer-Tropsch-Synthese zu einem Rohproduktstrom (synthetisches Rohöl) umgesetzt wird, der leichte Kohlenwasserstoffe mit vier oder weniger Kohlenstoffatomen, schwere Kohlenwasserstoffe mit fünf oder mehr Kohlenstoffatomen sowie nicht umgesetztes Synthesegas aufweist.

Hierbei ist bevorzugt vorgesehen, dass ein Restgas aufweisend leichte Kohlenwasserstoffe sowie nicht umgesetztes Synthesegas vom Rohproduktstrom bzw. vom erzeugten synthetischen Rohöl (auch als synthetic crude bezeichnet) abgetrennt wird und zumindest teilweise in die Fischer-Tropsch-Synthese als Einsatz zurückgeführt wird, wobei ein Teil dieses Restgases als Einsatz in die Dampfreformation bzw. partielle Oxidation zurückgeführt wird und/oder als Brennstoff verwendet wird.

Vorzugsweise ist weiterhin vorgesehen, dass Wasserstoff aus dem H₂-haltigen Strom, der bei der Druckwechseladsorption gewonnen wird, zur Hydrogenierung von schweren Kohlenwasserstoffen (z.B. Aromaten) und/oder Oxygenaten des Rohproduktstroms verwendet wird, wobei der Rohproduktstrom hiernach in einen oder mehrere kohlenwasserstoffhaltige Produktströme aufgeteilt wird.

Alternativ oder ergänzend kann das hergestellte Synthesegas gemäß einer weiteren Ausführungsform der Erfindung auch für eine Methanolsynthese verwendet werden.

Hierbei wird der erste Synthesegasteilstrom vorzugsweise in einer Methanolsynthese zu einem Methanol aufweisenden Rohproduktstrom umgesetzt, wobei bevorzugt im Rohproduktstrom enthaltenes Methanol vom im Rohproduktstrom enthaltenen, nicht umgesetzten Synthesegas unter Erzeugung eines Methanolproduktstroms abgetrennt wird, wobei das abgetrennte, nicht umgesetzte Synthesegas vorzugsweise als Einsatz in die Methanolsynthese zurückgeführt wird.

Im Fall einer Methanolsynthese kann natürlich der bei der Druckwechseladsorption gewonnene Wasserstoff auch als Wasserstoffprodukt bereitgestellt werden.

Unabhängig von der verwendeten Synthese stromab der Synthesegaserzeugung wird das in dem Synthesegaserzeugungsschritt erzeugte Synthesegas vorzugsweise mit Wasser gekühlt, wobei Wasserdampf erzeugt wird. Dieser wird vorzugsweise zur Erzeugung von elektrischer Energie verwendet, wobei bevorzugt der Wasserdampf zuvor in einem bei der besagten Dampfreformation verwendeten Ofen oder in einer sonstigen Verbrennungseinrichtung überhitzt wird.

Weitere Merkmale und Vorteile der Erfindung werden nachfolgend bei der Figurenbeschreibung von Ausführungsbeispielen der Erfindung anhand der Figuren erläutert. Es zeigen:
- Fig. 1: eine schematische Darstellung eines erfindungsgemäßen Verfahrens zur Herstellung eines Synthesegases aus einem CO₂-reichen Einsatzgas, wobei das hergestellte Synthesegas in einer Fischer-Tropsch-Synthese verwendet wird; und
- Fig. 2: eine schematische Darstellung eines erfindungsgemäßen Verfahrens zur Herstellung eines Synthesegases aus einem CO₂-reichen Einsatzgas, wobei das hergestellte Synthesegas in einer Methanolsynthese verwendet wird.

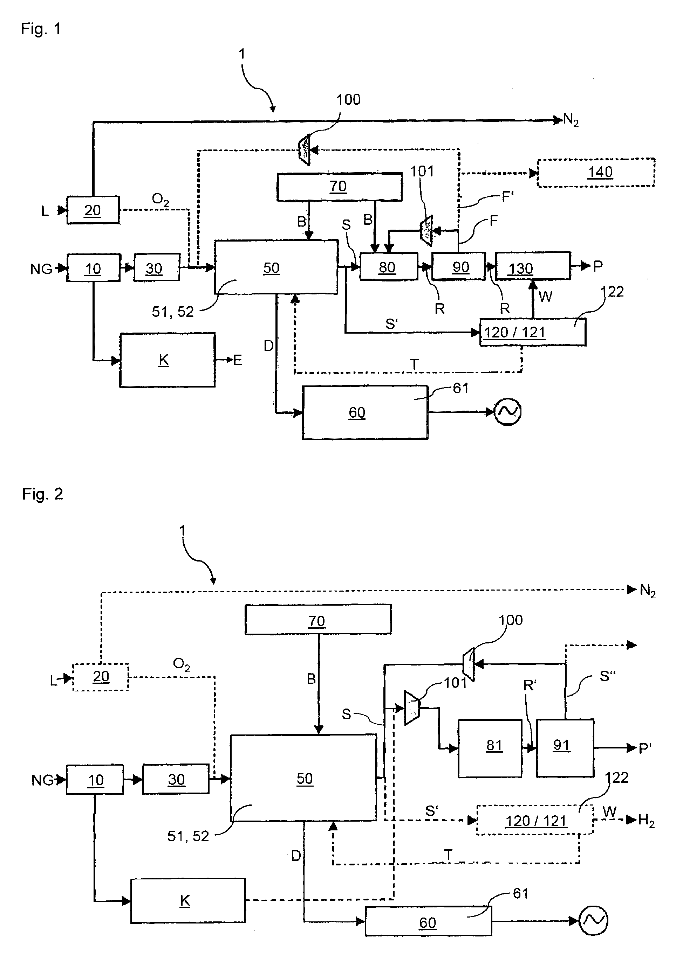

Figur 1 zeigt eine schematische Darstellung einer Anlage 1 bzw. eines Verfahrens Verfahren zur Erzeugung eines Synthesegases aus einem CO₂-reichen und kohlenwasserstoffhaltigen Einsatzgas NG, insbesondere Erdgas, das gemäß einem Beispiel neben CO₂ mit einem Gehalt von 10 Vol.-% bis 70 Vol.-% und ggf. einer oder mehrerer Schwefelverbindungen, wie z.B. H₂S, CS₂, COS und/oder HCN, mit je einem Gehalt im Bereich von bis zu 5 Vol.-%, zumindest einen der folgenden Kohlenwasserstoffe bzw. Stoffe aufweist: 25 Vol.-% bis 95 Vol.-% CH₄, 5 Vol.-% bis 75 Vol.-% CO₂, bis zu 5 Vol.-%: Ethan, bis zu 3 Vol.-% Propan, bis zu 2 Vol.-% Butan, bis zu 3 Vol.-% Pentan, bis zu 5 Vol.% Stickstoff.

Der Einsatzgasstrom NG wird vor einer Umsetzung zu Synthesegas (aufweisend H₂ und CO) erfindungsgemäß einer Wäsche unterzogen, um zumindest CO₂ und ggf. vorhandenen Schwefelkomponenten wie z.B. H₂S, CS₂, COS aus dem Einsatzgas NG zu entfernen. Bei dieser auch als Sauergaswäsche bezeichneten Wäsche 10 (insbesondere Rectisolverfahren) wird CO₂ sowie ggf. vorhandene Schwefelkomponenten bevorzugt wie oben beschrieben aus dem Einsatzgas NG abgetrennt, wobei bevorzugt CO₂ und jene Schwefelkomponenten separat abgetrennt werden. Hierdurch wird ein CO₂-reicher Strom bzw. ein CO₂-Strom K (insbesondere mit bis zu 75 Vol.-% CO₂) erzeugt, der einen Druck im Bereich von 15bar bis 100 bar aufweist.

Der CO₂-reiche Strom K kann z.B. als Einsatz für eine Synthese verwendet werden, z.B. für eine Methanolsynthese 81 gemäß Figur 2 oder z.B. zur Unterstützung der Förderung von Erdöl, wobei der CO₂-reiche Strom K z.B. in eine Erdöl-Lagerstätte E injiziert werden kann um den Lagerstättendruck zu erhöhen.

Stromab der Sauergaswäsche 10 wird der Einsatzgasstrom NG des Weiteren bevorzugt in einer Adsorbereinheit 30 von noch vorhandenen Spuren an CO₂ und/oder Schwefelverbindungen befreit, wobei der Gehalt an Schwefelkomponenten auf unter 10 ppb reduziert wird, beispielsweise mittels eines Guard-Betts.

Hiernach wird der Einsatzgasstrom in einem Synthesegaserzeugungsschritt 50 zu Synthesegas (enthaltend H₂ und CO) umgesetzt. Hierzu kann eine partielle Oxidation 50 oder eine Dampfreformation 50 verwendet werden.

Bei der Dampfreformation 50 wird der vorgereinigte Einsatzgasstrom NG wie oben beschrieben mit Wasserdampf vermischt und in Reaktorrohren, in denen ein geeigneter Katalysator angeordnet ist, bei einer Temperatur im Bereich von z.B. 700 °C bis 950 C sowie einem Druck im Bereich von z.B. 20 bar bis 50bar in Synthesegas umgesetzt, das sodann gekühlt und getrocknet wird.

Alternativ oder ergänzend kann auch eine partielle Oxidation 50 verwendet werden, bei der das Einsatzgas NG wie oben beschrieben mit Sauerstoff bei einer Temperatur im Bereich von z.B. 1100 °C bis 1300°C sowie einem Druck im Bereich von z.B. 20 bar bis 100 bar zu Synthesegas umgesetzt wird. Als Oxidationsmittel wird vorzugsweise reiner Sauerstoff verwendet, der durch kryogene Luftzerlegung 20 erzeugt wird und dem Einsatzgas NG stromab der Sauergaswäsche 10, stromab der Adsorbereinheit 30, sowie stromauf des Synthesegaserzeugungschritts 50 zugegeben wird.

Das erzeugte Synthesegas wird in einen ersten und einen zweiten Synthesegasteilstrom S, S' aufgeteilt, wobei der erste Synthesegasteilstrom S (85 bis 95 Vol.-%) als Einsatz einer Fischer-Tropsch-Synthese 80 zugeführt wird, und wobei der zweite Synthesegasteilstrom S' (5 bis 15 Vol.-%) einer Wassergas-Shift-Reaktion 120 unterzogen wird, bei der CO des zweiten Synthesegasteilstromes S' mit H₂O zu H₂ und CO₂ umgesetzt wird, um den CO-Gehalt im zweiten Synthesegasteilstrom S' zu verringern und den Wasserstoffgehalt im zweiten Synthesegasteilstrom S' zu erhöhen.

Nach der Wassergas-Shift-Reaktion 120 wird der zweite Synthesegasteilstrom S' einer bekannten Druckwechseladsorption 121 unterzogen, wobei im zweiten Synthesegasteilstrom S' enthaltenes CO₂ an zumindest einem Adsorber 122 bei einem ersten Druck (z.B. im Bereich von 15 bar bis 35 bar) sowie einer Temperatur im Bereich von 20 °C bis 80 °C adsorbiert wird und ein H₂-haltiger Strom W (insbesondere mit einem H₂-Gehalt von 85 bis 97% Vol.-%) erzeugt wird, und wobei der Adsorber 122 bei einem zweiten Druck (z.B. im Bereich von 20 bar bis 35 bar) sowie einer Temperatur im Bereich von 40°C bis 120°C regeneriert wird, wobei adsorbiertes CO₂ desorbiert wird, und wobei der Adsorber 122 zum Entfernen des desorbierten CO₂ (sowie ggf. weiterer desorbierter Komponenten) mit H₂ unter Erzeugung eines H₂-haltigen Spülgasstroms T gespült wird. Vorzugsweise werden mehrere, insbesondere zwei oder vier Adsorber bei der Druckwechseladsorption verwendet, damit möglichst immer ein Adsorber im Adsorptionsmodus betrieben werden kann, so dass quasi kontinuierlich Wasserstoff abgegeben werden kann. Der Spülgasstrom T kann z.B. als Brennstoff in einem Ofen 51 zur Durchführung der Dampfreformation 50 verbrannt werden und/oder als Brennstoff zum Erzeugen und/oder Überhitzen von Wasserdampf verwendet werden.

Bei der besagten Fischer-Tropsch-Synthese 80 wird der erste Synthesegasteilstrom S in bekannter Weise zu einem Rohproduktstrom R umgesetzt, der leichte Kohlenwasserstoffe mit vier oder weniger Kohlenstoffatomen, schwere Kohlenwasserstoffe mit fünf oder mehr Kohlenstoffatomen sowie nicht umgesetztes Synthesegas aufweiset. Für die Synthese 80 benötigtes Wasser B wird mittels einer Wasserversorgung 70 bereitgestellt. Vom Rohproduktstrom R wird ein Restgas F aufweisend die leichten Kohlenwasserstoffe sowie nicht umgesetztes Synthesegas abgetrennt, wobei zumindest ein Teil des Restgases F nach Verdichtung in einem Verdichter 101 (z.B. auf einen Druck im Bereich von 15 bar bis 35 bar) in die Fischer-Tropsch-Synthese 80 als Einsatz zurückgeführt wird. Ein weiterer Teil F' des Restgases F kann nach Verdichtung in einem Verdichter 100 (z.B. auf einen Druck im Bereich von 20 bar bis 50 bar) als Einsatz in die Dampfreformation 50 zurückgeführt werden und/oder als Brennstoff 140 verwendet werden.

Der bei der Druckwechseladsorption 121 erzeugte H₂-haltige Strom W wird des Weiteren z.B. zur Hydrogenierung 130 von schweren Kohlenwasserstoffen des Rohproduktstroms R der Fischer-Tropsch-Synthese verwendet. Der aufbereitete Rohproduktstrom R wird in einen oder mehrere kohlenwasserstoffhaltige Produktströme P aufgeteilt, die unterschiedliche Kohlenwasserstofffraktionen aufweisen können.

Fig. 2 zeigt ein weiteres Ausführungsbeispiel der Erfindung, bei der im Unterschied zur Fig.1 keine Fischer-Tropsch-Synthese durchgeführt wird, sondern eine Methanolsynthese 81. Hierbei wird der erste Synthesegasteilstrom S in einem Verdichter 101 verdichtet (z.B. auf einen Druck im Bereich von 20 bar bis 100 bar) und in der Methanolsynthese 81 zu einem Methanol aufweisenden Rohproduktstrom R' umgesetzt, wobei im Rohproduktstrom R' enthaltenes Methanol vom im Rohproduktstrom R' enthaltenen, nicht umgesetzten Synthesegas S" getrennt wird 91, wobei ein Methanolproduktstrom P' erzeugt wird Das abgetrennte, nicht umgesetzte Synthesegas S" wird in einem Verdichter 100 verdichtet (z.B. auf einen Druck im Bereich von 40 bar bis 100 bar) und als Einsatz in die Methanolsynthese 81 zurückgeführt und zwar über den anderen Verdichter 101 (wobei insbesondere eine Druckerhöhung auf einen Druck im Bereich von 40 bar bis 100 bar erfolgt). Der bei der Druckwechseladsorption 121 gewonnene H₂-haltige Strom W kann z.B. als Wasserstoffprodukt bereitgestellt werden.

Weiterhin kann bei der Ausführungsform gemäß Fig. 2 auch der CO₂-reiche Strom K als Einsatz für die Methanolsynthese 81 verwendet werden (vgl. Fig. 1), wobei der Strom K über den Verdichter 101 in die Methanolsynthese geführt wird und dabei auf einen Druck im Bereich von 40 bar bis 100 bar verdichtet wird.

In den Ausführungsformen gemäß Figur 1 und 2 wird jeweils das in dem Synthesegaserzeugungsschritt 50 erzeugte Synthesegas mit Wasser B der Wasserversorgung 70 gekühlt, wobei Wasserdampf D erzeugt wird, der zur Erzeugung von elektrischer Energie 60 verwendet werden kann. Hierbei kann der Wasserdampf D z.B. in dem Ofen 51 der Dampfreformation 50 oder in einem sonstigen Verbrennungsofen 52 überhitzt und sodann zur Erzeugung elektrischer Energie verwendet werden, z.B. in einer Dampfturbine 61.

Im Ergebnis ermöglicht es die erfindungsgemäße Lehre, einen vergleichsweise niedrigen Inerten- bzw. CO₂-Gehalt im Synthesegasstrom zu erhalten, wobei die Anlage insgesamt kleiner ausfallen kann, mit einem niedrigeren Energieverbrauch auskommt und die zu rückzuführenden Prozessströme mit Vorteil vergleichsweise kleiner ausfallen. Bei der POX wird ein geringerer Sauerstoffverbrauch möglich.

**Bezugszeichenliste**

| | |
|---|---|
| 1 | Anlage zur Synthesegasherstellung sowie Synthese von Kohlenwasserstoffen |
| 10 | Wäsche zur CO₂-Entfernung |
| 20 | Luftzerlegungseinheit |
| 30 | Adsorbereinheit zur Entschwefelung |
| 50 | Synthesegaserzeugungsschritt sowie Synthesegaskühlung |
| 51 | Ofen für Dampfreformation |
| 52 | Verbrennungsofen |
| 60 | Energieerzeugung |
| 61 | Dampfturbine |
| 70 | Wasserversorgung |
| 80 | Fischer-Tropsch-Synthese |
| 81 | Methanolsynthese |
| 90, 91 | Abtrennung |
| 100, 101 | Verdichter |
| 120 | Wassergas-Shift-Reaktion |
| 121 | Druckwechseladsorption |
| 130 | Produktaufarbeitung |
| 140 | Brennstoff-System bzw. -Versorgung |
| B | Wasser |
| E | Erdöllagerstätte |
| F, F' | Reststrom |
| K | CO₂-reicher Strom |
| L | Luft |
| NG | Einsatzgas |
| P, P' | Produktstrom |
| R, R' | Rohproduktstrom |
| S | Erster Synthesegasteilstrom |
| S' | Zweiter Synthesegasteilstrom |
| S" | Nicht umgesetztes Synthesegas |
| T | Spülgas |
| W | Wasserstoffhaltiger Strom |

## Patentansprüche

1. Verfahren zur Erzeugung eines Synthesegases aus einem CO₂-reichen und kohlenwasserstoffhaltigen Einsatzgas (NG), wobei ein CO₂-reiches und kohlenwasserstoffhaltiges Einsatzgas (NG) bereitgestellt wird und in einem Synthesegaserzeugungsschritt (50) mittels partieller Oxidation (50) und/oder Dampfreformierung (50) zu einem H₂ und CO aufweisenden Synthesegas umgesetzt wird,
**dadurch gekennzeichnet, dass**
zumindest CO₂ aus dem Einsatzgas (NG) bei einer Wäsche (10) des Einsatzgases mittels eines Waschmittels entfernt wird, bevor das Einsatzgas (NG) dem Synthesegaserzeugungsschritt (50) zugeführt wird, wobei bei der Wäsche (10) ein CO₂-reicher Strom (K) erzeugt wird, der einen Druck im Bereich von 20 bar bis 100 bar aufweist, und wobei der CO₂-reiche Strom als Einsatz für eine Synthese verwendet wird und/oder zur Unterstützung der Förderung von Erdöl, wobei der CO₂-reiche Strom (K) in eine Erdöl-Lagerstätte (E) injiziert wird, um den Druck in der Erdöl-Lagerstätte (E) zu erhöhen.

2. Verfahren nach einem der vorhergehenden Ansprüche, **dadurch gekennzeichnet, dass** das Einsatzgas (NG) stromab der Wäsche (10) durch eine Adsorbereinheit (30) geführt wird, wobei eine oder mehrere noch im Einsatzgas (NG) befindliche Schwefelverbindungen in der Adsorbereinheit (30) adsorbiert und dabei aus dem Einsatzgas (NG) entfernt werden.

3. Verfahren nach einem der vorhergehenden Ansprüche, **dadurch gekennzeichnet, dass** das erzeugte Synthesegas (50) in einen ersten und einen zweiten Synthesegasteilstrom (S, S') aufgeteilt wird, wobei der erste Synthesegasteilstrom (S) als Einsatz für eine Synthese (80, 81) verwendet wird, und wobei der zweite Synthesegasteilstrom (S') einer Wassergas-Shift-Reaktion (120) unterzogen wird, wobei CO des zweiten Synthesegasteilstromes (S') mit H₂O zu H₂ und CO₂ umgesetzt wird, um den CO-Gehalt im zweiten Synthesegasteilstrom (S') zu verringern und den Wasserstoffgehalt im zweiten Synthesegasteilstrom (S') zu erhöhen.

4. Verfahren nach einem der vorhergehenden Ansprüche, **dadurch gekennzeichnet, dass** die Reduktion des CO₂-Gehalts im Einsatzgas (NG) bei der Wäsche (10) in Abhängigkeit von einer stromab der Synthesegaserzeugung (50) vorgesehenen Verwendung des Synthesegases (80, 81) und/oder in Abhängigkeit eines gewünschten Verhältnisses von CO zu H₂ im Synthesegas eingestellt wird.

5. Verfahren nach Anspruch 3, **dadurch gekennzeichnet, dass** der zweite Synthesegasteilstrom (S') nach der Wassergas-Shift-Reaktion (120) einer Druckwechseladsorption (121) unterzogen wird, wobei im zweiten Synthesegasteilstrom (S') enthaltenes CO₂ an einem Adsorber (122) bei einem ersten Druck adsorbiert wird und ein H₂-haltiger Strom (W) erzeugt wird, und wobei der Adsorber (122) bei einem zweiten Druck, der niedriger ist als der erste Druck, regeneriert wird, wobei adsorbiertes CO₂ desorbiert wird, und wobei der Adsorber (122) zum Entfernen des desorbierten CO₂ mit H₂ unter Erzeugung eines H₂-haltigen Spülgasstroms (T) gespült wird.

6. Verfahren nach Anspruch 5, **dadurch gekennzeichnet, dass** der Spülgasstrom (T) als Brennstoff verwendet wird, wobei das Spülgas (T) in einem Ofen (51) zur Durchführung der Dampfreformation (50) verbrannt wird und/oder wobei das Spülgas (T) in einem Verbrennungsofen (52) zum Erzeugen und/oder Überhitzen von Wasserdampf verbrannt wird.

7. Verfahren nach einem der vorhergehenden Ansprüche, **dadurch gekennzeichnet, dass** Sauerstoff kryogen aus Luft (L) abgetrennt wird (20) und als Oxidationsmittel bei der partiellen Oxidation (50) verwendet wird, wobei der Sauerstoff dem Einsatzgas (NG) stromab der Wäsche (10), stromab der Adsorbereinheit (30) sowie stromauf des Synthesegaserzeugungschritts (50) dem Einsatzgas (NG) zugegeben wird.

8. Verfahren nach Anspruch 3 oder einem der Ansprüche 4 bis 7 soweit rückbezogen auf Anspruch 3, **dadurch gekennzeichnet, dass** die Synthese eine Fischer-Tropsch-Synthese ist (80), wobei der erste Synthesegasteilstrom (S') in der Fischer-Tropsch-Synthese (80) zu einem Rohproduktstrom (R) umgesetzt wird, der leichte Kohlenwasserstoffe mit vier oder weniger Kohlenstoffatomen, schwere Kohlenwasserstoffe mit fünf oder mehr Kohlenstoffatomen sowie nicht umgesetztes Synthesegas aufweist.

9. Verfahren nach Anspruch 8, **dadurch gekennzeichnet, dass** ein Restgas (F) aufweisend leichte Kohlenwasserstoffe sowie nicht umgesetztes Synthesegas vom Rohproduktstrom (R) abgetrennt wird (90) und zumindest teilweise in die Fischer-Tropsch-Synthese (80) als Einsatz zurückgeführt wird, wobei ein Teil (F') des Restgases (F) als Einsatz in die Dampfreformation (50) und/oder partielle Oxidation (50) zurückgeführt wird und/oder als Brennstoff verwendet wird (140).

10. Verfahren nach Anspruch 5 und 9, **dadurch gekennzeichnet, dass** Wasserstoff aus dem H₂-haltigen Strom (W) zur Hydrogenierung von schweren Kohlenwasserstoffen des Rohproduktstroms (R) verwendet wird, wobei der Rohproduktstrom (R) hiernach in einen oder mehrere kohlenwasserstoffhaltige Produktströme (P) aufgeteilt wird.

11. Verfahren nach Anspruch 3 oder einem der Ansprüche 4 bis 7 soweit rückbezogen auf Anspruch 3, **dadurch gekennzeichnet, dass** die Synthese eine Methanolsynthese (81) ist, wobei der erste Synthesegasteilstrom (S) in der Methanolsynthese (81) zu einem Methanol aufweisenden Rohproduktstrom (R') umgesetzt wird.

12. Verfahren nach Anspruch 11, **dadurch gekennzeichnet, dass** im Rohproduktstrom (R') enthaltenes Methanol vom im Rohproduktstrom (R') enthaltenen, nicht umgesetzten Synthesegas (S") unter Erzeugung eines Methanolproduktstroms (P') getrennt wird (91), wobei das abgetrennte, nicht umgesetzte Synthesegas (S") als Einsatz in die Methanolsynthese (81) zurückgeführt wird.

13. Verfahren nach einem der vorhergehenden Ansprüche, **dadurch gekennzeichnet, dass** das in dem Synthesegaserzeugungsschritt (50) erzeugte Synthesegas mit Wasser (B) gekühlt wird, wobei Wasserdampf (D) erzeugt wird, der zur Erzeugung von elektrischer Energie verwendet wird (60), wobei der Wasserdampf (D) in einem Ofen (51) zur Durchführung der Dampfreformation (50) oder in einem sonstigen Verbrennungsofen (52) überhitzt wird und sodann zur Erzeugung elektrischer Energie in einer Dampfturbine (61) verwendet wird.
